# EUROPÄISCHE PATENTSCHRIFT

(11) **EP 0 634 109 B1**
(45) Veröffentlichungstag und Bekanntmachung des Hinweises auf die Patenterteilung: **20.09.2000**
(21) Anmeldenummer: 94109609.1
(22) Anmeldetag: 22.06.1994
(51) Int. Cl.: A23L 2/38, A23L 1/304

(54) **Feste Zusammensetzung für ein Mineralstoffgetränk**
Solid composition for a mineral drink
Composition solide pour boisson minérale

(30) Priorität: 23.06.1993 DE 4320853
(43) Veröffentlichungstag der Anmeldung: 18.01.1995
(73) Patentinhaber: Schwille, Paul Otto, Prof. Dr. Dr., 91080 Uttenreuth (DE)
(72) Erfinder: Schwille, Paul Otto, Prof. Dr. Dr., 91080 Uttenreuth (DE)
(74) Vertreter: Pfenning, Meinig & Partner

(56) Entgegenhaltungen:
- EP-A- 0 040 654
- WO-A-85/02324
- FR-A- 2 107 331
- GB-A- 1 252 781
- "Taschenbuch der Chemie" von W. Schröter, Dr. Lautenschläger und H. Bibrack, 9. Auflage, ISBN 3-87 144-308-5, Verlag Harri Deutsch (FfM), S. 213-216 : "die Säure-Base-Definition Brönstedts"

## Beschreibung

Die Erfindung betrifft eine feste Zusammensetzung für ein Mineralstoffgetränk und ein daraus hergestelltes Mineralstoffgetränk, enthaltend Magnesium, Kalium und Natrium in wasserlöslicher Form.

Die heutige Ernährung der Menschen in den hochindustrialisierten Ländern ist nicht nur reich an Energie (Zufuhr von Kalorien), sondern führt regelmäßig zuviel an Nahrungsgrundbestandteilen zu, deren Abbau im Organismus einen Überhang an Säuren (sogenannte saure Asche) zurückläßt. Unter solche Nahrungsbestandteile fallen vor allem tierisches Eiweiß, wie Muskelfleisch, Innereien und Fisch. Die Ausscheidung der Endprodukte des Stoffwechsels dieser Eiweiße verbraucht dabei einen hohen Anteil der im Körper rasch verfügbaren Puffersubstanzen. Hinzu kommt ein damit einhergehender unverhältnismäßig hoher Verlust, vor allem über den Urin, an lebensnotwendigen Mineralien, wie Magnesium, Kalium, Calcium, zu einem geringen Grad auch Natrium. Auf lange Sicht führt diese doppelte Belastung - defizitäre Zufuhr an Basen und gesteigerter Verlust von Mineralien - zu unerwünschten Nebenwirkungen an verschiedenen Geweben, wie Niere, Blutgefäße, Knochen. Unter den Folgen dieser Fehlernährung sind vermehrte Bildung von Harnsteinen und Zunahme der Verengung von Blutgefäßen.

Voraussetzung für die Aufrechterhaltung normaler biologischer Vorgänge im menschlichen Organismus sind ausreichende Konzentrationen an Basen, Magnesium und Kalium. Wegen der unterschiedlichen Wertigkeiten von Mg und K ist ein Verhältnis von 1:2 bis 1:3 anzustreben. Es sind zwar bereits verschiedene Mineralgetränke bekannt, welche besonders ein auf die vorgenannte Weise zustande gekommenes Defizit an Magnesium im Körper ausgleichen sollen. Diese Getränke enthalten jedoch Magnesium in einer Salzform, welche für sich allein nicht geeignet ist, die begleitende Überproduktion von Säure abzupuffern. In den allermeisten fehlt Kalium völlig. In einigen ist ein Anteil Natrium enthalten, als Bicarbonat oder Carbonat; die davon abzuleitende basische Wirkung ist quantitativ ungenügend. Höhere Anteile Natrium führen zur unerwünschten Steigerung von Calciumverlust über den Urin.

Aus dem Stand der Technik ist auch die EP-A-0040654 bekannt, die ein konzentriertes Mineralstoffgetränk offenbart, das u.a. Natrium-, Kalium-, Magnesiumionen und Citrat enthält.

Hiervon geht die vorliegende Erfindung aus, deren Aufgabe es ist, ein Mineralgetränk vorzuschlagen, das nicht nur Magnesium und andere lebenswichtige Mineralien zuführt, sondern gleichzeitig ermöglicht, die Überproduktion von Säure abzupuffern.

Die Erfindung wird hinsichtlich der festen Mineralstoffmischung durch die kennzeichnenden Merkmale des Anspruchs 1 und bezüglich des Mineralgetränks durch die Merkmale des Anspruchs 8 gelöst. Die Unteransprüche geben vorteilhafte Weiterbildungen an.

Das aus der erfindungsgemäßen festen Mineralstoffmischung hergestellte Mineralgetränk zeichnet sich besonders dadurch aus, daß es neben den Mineralien Magnesium, Kalium und Natrium auch Basen zur Pufferung von Säure enthält. Erfindungsgemäß wird dies dadurch erreicht, daß als Salze Citrate und/oder Bicarbonate verwendet werden. Von diesen Salzen ist bekannt, daß sie sich auch in kaltem Wasser auflösen und vollständig dissoziieren. Die Zusammensetzung enthält in 3 - 4 Gew.-% Mg, 14 - 17 Gew.-% K und 2,5 - 3,5 Gew.-% Na. In Wasser vollständig lösliche Salze organischer Säuren werden im Körper zu Basen-Äquivalenten abgebaut. Weil in der Mischung solche Salze vorliegen, wird erreicht, daß wenn die feste Mineralstoffmischung in Wasser aufgelöst und getrunken wird, dem Körper ein Mineralgetränk zur Verfügung steht, das neben den lebenswichtigen Mineralien genügend Base liefert, um die durch Überproduktion hervorgerufene Säure abzupuffern.

Vorteilhaft ist dabei für Mg die Verwendung von löslichem Monomagnesiumhydrogencitrat. Bevorzugt ist es weiterhin, wenn auch die anderen in der testen Mineralstoffmischung enthaltenen Mineralien in Form von wasserlöslichen Salzen vorliegen, die nach der Dissoziation eine Base bilden. Besonders bevorzugt ist dabei der Einsatz von Kalium als Citrat und Bicarbonat, in Kombination mit einem kleineren Anteil Natrium als Bicarbonat.

Eine bevorzugte Ausführungsform sieht nun vor, daß Kalium in Form von Kaliumbicarbonat und Trikaliumcitrat sowie Natrium in Form von Natriumbicarbonat eingesetzt wird. Besonders wichtig bei der erfindungsgemäßen Mineralstoffmischung ist, daß 3 - 4 Gew.-% Magnesium, 14 - 17 Gew.-% Kalium und 2,5 - 3,5 Gew.-% Natrium, jeweils berechnet auf den Gehalt am Element, in Form der wasserlöslichen Salze eingesetzt werden.

Bei durchgeführten Untersuchungen hat sich gezeigt, daß, wenn die vorstehend genannten Bereichsangaben der mengenmäßigen Zusammensetzungen verlassen werden, ein starker Verlust an biologischen Wirkungen des Substanzgemisches eintritt oder unerwünschte Wirkungen auftreten.

Durch zahlreiche Versuche an gesunden Menschen konnte die Anmelderin nämlich zeigen, daß durch die erfindungsgemäße Mineralstoffmischung, wenn sie in Form eines Mineralgetränks nach Auflösen in Wasser vom menschlichen Organismus aufgenommen wird, eine starke Alkalisierung des Stoffwechsels eintritt. Die Alkalisierung des Stoffwechsels wurde dabei nachgewiesen durch einen Abfall von sogenannter titrierbarer Säure und Ammonium im Urin.

Gleichzeitig konnte festgestellt werden, daß eine Einbehaltung eines vergleichsweise hohen Prozentsatzes von zugeführtem Magnesium durch den Körper und eine Zunahme von Citrat im Urin eintritt.

Bei regelmäßiger Anwendung der erfindungsgemäßen Mineralfeststoffmischung in Form des Mineralgetränks ist deshalb zu erwarten, daß einer Verkalkung in Nieren und Arterien entgegengewirkt wird; dies wäre gleichbedeutend mit einer Verzögerung natürlich ablaufender Alterungsprozesse.

Wie bereits vorstehend ausgeführt, wird die erfindungsgemäße feste Mineralstoffmischung in der Weise angewandt, daß sie in Wasser aufgelöst und dann durch Trinken dem menschlichen Organismus zugeführt wird. Es ist dabei bevorzugt, wenn ca. 2 - 8 g der vorstehend beschriebenen Mineralstoffmischung in 100 - 200 ml Wasser aufgelöst werden.

Wenn z.B. 5 g der Mineralstoffmischung in ca. 150 ml Wasser eingebracht wird, entwickelt sich Gas (CO₂) über max. ca. 2 Minuten, was die für die Präparation festgelegte Geschmacks- und Geruchsrichtung (Orange, Grapefruit etc.) vorteilhaft zum Ausdruck bringt.

Das Mineralstoffgetränk eignet sich deshalb besonders gut als Anti-Schlacke-Drink. Der Anti-Schlacke-Drink, der aus der erfindungsgemäßen festen Mineralstoffmischung hergestellt wird, unterstützt demnach aufgrund seines Gehalts an Basen und Mineralien die Aufrechterhaltung normaler biologischer Vorgänge im Körper, insbesondere die Funktion von Nieren, Blutgefäßen und Muskeln.

Der Anti-Schlacke-Drink sorgt zusätzlich für Wohlbefinden während außerordentlicher Belastung (Schwangerschaft, Stillzeit, Schwerarbeit, Fastenkuren oder Extremsportarten), bzw. im Anschluß an diese für rasche Normalisierung des Stoffwechsels. Allgemein kann gesagt werden, daß der aus der erfindungsgemäßen Mineralstoffmischung hergestellte Anti-Schlacke-Drink allgemein die Entwicklung von körperlichen und geistigen Kräften fördert. Der Anti-Schlacke-Drink ist zudem kalorienarm und billig in der Herstellung. Der Anti-Schlacke-Drink erfüllt demnach in idealer Weise alle Voraussetzungen, welche von einem Nahrungsergänzungsmittel bzw. diätetischem Lebensmittel verlangt werden. Er enthält ausschließlich Grundbestandteile der Nahrung (Mineralien, Basen, Vitamine), alle in vollständig löslicher und deshalb im Darm rasch aufnehmbarer Form. Diese Eigenschaften des Anti-Schlacke-Drinks empfehlen deshalb seine Aufnahme in ärztliche Diätanweisungen.

Die Erfindung wird durch nachstehendes Ausführungsbeispiel näher erläutert:

Beispielhafte Rezeptur:

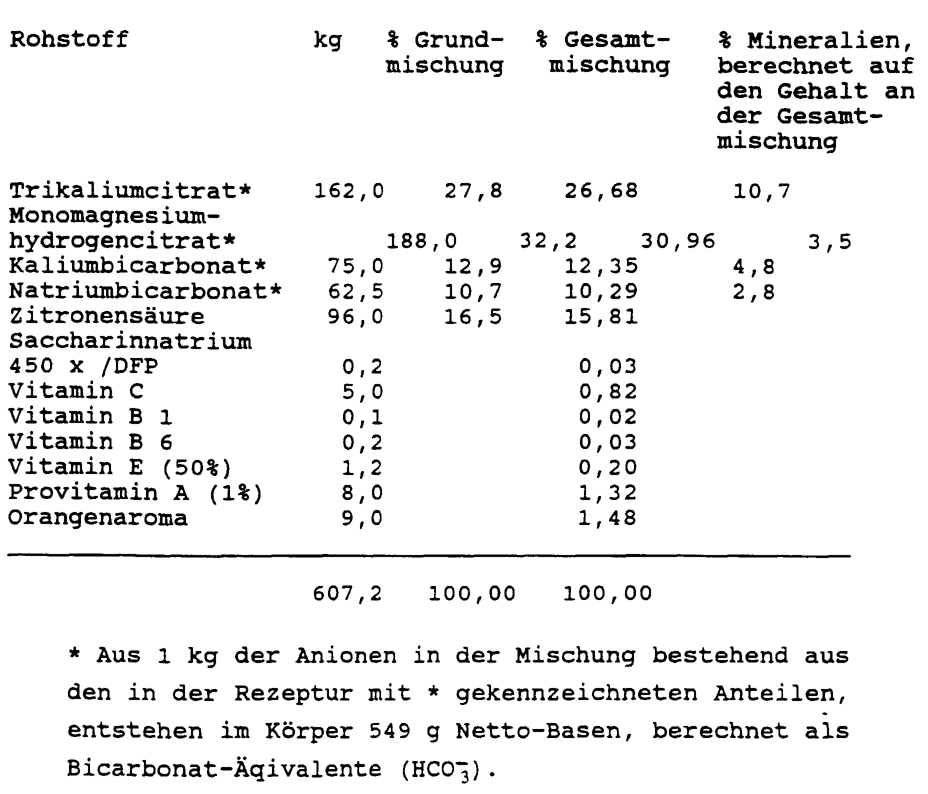

Von dieser Gesamtmischung werden nun zur Anwendung ca. 5g in ca. 150 ml Wasser aufgelöst. Für die Anwendung ist es deshalb vorteilhaft, 5g-Packungen mit der Mineralstoffmischung in den Handel zu bringen.

Die feste Mineralstoffmischung löst sich dabei in ca. 2 Min. nahezu vollständig auf. Nach dem Trinken auf nüchternen oder auch nur weitgehend leeren Magen tritt binnen Minuten eine vollständige Auflösung ein. Nach Vermischen der Mineralstoffmischung mit zusätzlicher im Magen und oberen Dünndarm anwesender Flüssigkeit, z.B. ca. 150 ml, entsteht eine Konzentration an osmotisch wirksamen Teilchen in der Größenordnung 200 - 300 mOsm/kg (entsprechend ca. 100 mOsm/0,3 kg). Bei dieser Situation werden Flüssigkeit (Wasser) und darin gelöste Bestandteile (Salze etc.) aus dem Darm in das Blut aufgenommen.

Bei Verbrauch von 1-2 Portionen zu je 5 - 6 g pro Tag und Person entstehen dabei Kosten, welche in der Größenordnung von 0,20 bis 0,50 DM pro Tag liegen.

## Patentansprüche

1. Feste Zusammensetzung für ein Mineralstoffgetränk enthaltend Magnesium-, Natrium- und Kaliumsalze sowie Zusatzstoffe,
dadurch **gekennzeichnet,**
daß sie 3 - 4 Gew.-% Mg, 14 - 17 Gew.-% K und 2,5 - 3,5 Gew.-% Na enthält, jeweils berechnet auf dem Gehalt an Elementen und daß die Salze Citrat und/oder Bicarbonat sind und zu 100 Gew.-% ergänzten Anteilen üblicher Zusatz- und Hilfsstoffe, wie z.B. Citronensäure, Vitamine, Aromen und Süßstoffe.

2. Feste Zusammensetzung für ein Mineralstoffgetränk nach Anspruch 1, dadurch gekennzeichnet, daß das Magnesiumsalz Monomagnesiumcitrat ist.

3. Feste Zusammensetzung für ein Mineralstoffgetränk nach Anspruch 1 oder 2, dadurch gekennzeichnet, daß das Kaliumsalz Trikaliumcitrat und/oder Kaliumbicarbonat ist.

4. Feste Zusammensetzung für ein Mineralstoffgetränk nach mindestens einem der Ansprüche 1 bis 3, dadurch gekennzeichnet, daß das Natriumsalz Natriumbicarbonat ist.

5. Feste Zusammensetzung für ein Mineralstoffgetränk nach mindestens einem der Ansprüche 1 bis 4, dadurch gekennzeichnet, daß die Vitamine oder deren Mischungen ausgewählt sind aus der Gruppe Vitamin C, Vitamin B1, Vitamin B6, Vitamin E, oder Provitamin A.

6. Feste Zusammensetzung für ein Mineralstoffgetränk nach mindestens einem der Ansprüche 1 bis 5, dadurch gekennzeichnet, daß der Süßstoff Saccharin ist.

7. Feste Zusammensetzung für ein Mineralstoffgetränk nach mindestens einem der Ansprüche 1 bis 7, dadurch gekennzeichnet, daß das Aroma Orangenaroma ist.

8. Mineralstoffgetränk, dadurch gekennzeichnet, daß 2 - 8 g der festen Zusammensetzung nach Anspruch 1 bis 7 in 100 - 200 ml Wasser gelöst ist.

9. Verwendung des Mineralstoffgetränks nach Anspruch 8, zur Herstellung eines Anti-Schlacke-Drinks.

## Claims

1. Solid composition for a mineral drink comprising salts of magnesium, of sodium and of potassium and also additives, characterized in that the composition comprises 3 - 4% by weight of Mg, 14 - 17% by weight of K and 2.5 - 3.5% by weight of Na, in each case calculated on the element content, and in that the salts are citrate and/or bicarbonate and contents, which are made up to 100% by weight, of customary additives and aids, for example citric acid, vitamins, flavourings and sweeteners.

2. Solid composition for a mineral drink according to Claim 1, characterized in that the magnesium salt is monomagnesium citrate.

3. Solid composition for a mineral drink according to Claim 1 or 2, characterized in that the potassium salt is tripotassium citrate and/or potassium bicarbonate.

4. Solid composition for a mineral drink according to at least one of Claims 1 to 3, characterized in that the sodium salt is sodium bicarbonate.

5. Solid composition for a mineral drink according to at least one of Claims 1 to 4, characterized in that the vitamins or their mixtures are selected from the group consisting of vitamin C, vitamin B1, vitamin B6, vitamin E, or provitamin A.

6. Solid composition for a mineral drink according to at least one of Claims 1 to 5, characterized in that the sweetener is saccharin.

7. Solid composition for a mineral drink according to at least one of Claims 1 to 7, characterized in that the flavouring is orange flavouring.

8. Mineral drink, characterized in that 2 - 8 g of the solid composition according to Claim 1 to 7 are dissolved in 100 - 200 ml of water.

9. Use of the mineral drink according to Claim 8 for producing a purifying drink.

## Revendications

1. Composition solide pour une boisson minérale, contenant des sels de magnésium, sodium et potassium ainsi que des additifs, caractérisée en ce qu'elle contient 3-4 % en poids de Mg; 14-17 % en poids de K et 2,5-3,5 % en poids de Na, dans chaque cas calculé sur la base de la teneur en éléments, et en ce que les sels sont des citrates et/ou bicarbonates, et des proportions complétant à 100 % en poids d'additifs et adjuvants usuels, comme par exemple l'acide citrique, des vitamines, des arômes et des édulcorants.

2. Composition solide pour une boisson minérale selon la revendication 1, caractérisée en ce que le sel de magnésium est le citrate de monomagnésium.

3. Composition solide pour une boisson minérale selon la revendication 1 ou 2, caractérisée en ce que le sel de potassium est le citrate tripotassique et/ou le bicarbonate de potassium.

4. Composition solide pour une boisson minérale selon au moins l'une des revendications 1 à 3, caractérisée en ce que le sel de sodium est le bicarbonate de sodium.

5. Composition solide pour une boisson minérale selon au moins l'une des revendications 1 à 4, caractérisée en ce que les vitamines ou mélanges de celles-ci sont choisis parmi la vitamine C, la vitamine B1, la vitamine B6, la vitamine E et la provitamine A.

6. Composition solide pour une boisson minérale selon au moins l'une des revendications 1 à 5, caractérisée en ce que l'édulcorant est la saccharine.

7. Composition solide pour une boisson minérale selon au moins l'une des revendications 1 à 7, caractérisée en ce que l'arôme est l'arôme orange.

8. Boisson minérale caractérisée en ce que 2-8 g de la composition solide selon l'une des revendications 1 à 7 sont dissous dans 100-200 ml d'eau.

9. Utilisation de la boisson minérale selon la revendication 8, pour la préparation d'une boisson contre les produits néfastes du métabolisme.
